Europäisches Patentamt

European Patent Office

Office européen des brevets

(11) Publication number: **0 451 436 A2**

# EUROPEAN PATENT APPLICATION

(21) Application number: **91100018.0**

(22) Date of filing: **11.03.91**

(51) Int. Cl.5: **A61K 37/64**, A61K 35/78, A61K 31/715

(30) Priority: **06.04.90 JP 90282/90**

(43) Date of publication of application:
**16.10.91 Bulletin 91/42**

(84) Designated Contracting States:
**DE FR GB IT SE**

(71) Applicant: **Kyodo Milk Industry Corporation Limited**
**17-2, Koami-cho Nihonbashi**
**Chuo-ku Tokyo(JP)**

(72) Inventor: **Oishi, Hifumi**
**No. 3O3, 27-6, Sekimachi-higashi 1-chome**
**Nerima-ku, Tokyo(JP)**
Inventor: **Hattori, Takashi**
**No. 205, 1-19, Jyosuihoncho 5 chome**
**Kodaira-shi, Tokyo(JP)**
Inventor: **Watanabe, Masatoshi**
**5-36, Midoricho 2-chome**
**Koganei-shi, Tokyo(JP)**
Inventor: **Kato, Akio**
**24-28, Kitamachi 4-chome**
**Kokubunji-shi, Tokyo(JP)**

(74) Representative: **Lesser, Karl-Bolko, Dipl.-Ing.**
**Patentanwalt & European Patent Attorney**
**Gnesener Strasse 2**
**W-8068 Pfaffenhofen 1(DE)**

(54) Method of curing and preventing obesity by alpha-amylase inhibitor.

(57) A method of curing and preventing obesity comprises orally administrating $\alpha$-amylase inhibitor thereby inhibiting an $\alpha$-amylase activity in saliva and pancreatic juice, and reducing digestion and absorption of starch (reduction of the calorie taken from meals). Obesity can be cured or prevented effectively while taking usual diet without giving any physical or mental pain.

EP 0 451 436 A2

Background of the invention

Field of the invention

The present invention concerns with a method of curing or preventing obesity by inhibiting $\alpha$-amylase activity in saliva and pancreatic juice in a living body and reducing the digestion and absorption of starch, thereby decreasing the total calorie intake from meals.

Description of the prior art

Since obesity is one of main causes for adult diseases or like other various diseases, various methods have been proposed or practiced for the cure and the prevention of obesity. However, since the total calorie is restricted in any of such methods by diet, which enforces physical and mental pains, and the aimed purpose cannot be satisfied in most cases. In addition, excess restriction of meals causes lack of essential nutrients and micronutrients, to rather injure health.

It has been, accordingly, desired for the development of a method capable of curing and preventing obesity, by removing only the excess calorie though the least means and improving the staying time of feces in intestines while taking usual diet without enforcing physical or mental pains.

For dissolving the above-mentioned problem, it is most effective to hinder the digestion and absorption of starch which constitutes about 24 % of the total calorie is taken by Japanese.

Summary of the invention

In view of the above, the present invention proposes a method of inhibiting the $\alpha$-amylase activity in saliva and pancreatic juice decomposing starch in the meals by using a specific inhibitor, thereby curing or preventing obesity.

As a result of using the specific inhibitor, since starch in food-stuffs passes digestive tracts in a state in which it is not decomposed or decomposed only insufficiently, due to enzymes in digestive juices absorption in the digestive tracts is hindered. In this case, it is caused neither excess increase for the absolute mol concentration of the resultant not-decomposed starch nor excess increase in the mol concentration in the digestive tracks in a case of using an inhibitor to maltase, invertase or like other enzyme which would bring about uncomfortable feeling due to caddy stool, diarrhea and satiety. In addition, the not-decomposed starch exhibits a so-called fiber effect to increase the amount of stool and improve its staying time in intestines.

Example

Several kinds of experiments were conducted for the hindrance of intestinal absorption of starch by oral administration of $\alpha$-amylase inhibitors derived from soy-bean and wheat, as well as the effects thereof and description will then be made to them.

(1) The activity of $\alpha$-amylase inhibitors obtained from soy-bean and wheat against $\alpha$-amylase derived from human saliva (Sigma Co.), $\alpha$-amylase derived from mouse saliva (Sigma Co.) and $\alpha$-amylase derived from mouse pancreas was examined. Each of $\alpha$-amylases was dissolved in TBS (Tris-HCl buffer saline, 2 units/ml, TBS). Each of $\alpha$-amylase inhibitors was also dissolved in the same buffer (5 mg/ml, TBS), and each 100 ml solution of $\alpha$-amylase and $\alpha$-amylase inhibitor were brought into reaction at 37 $^\circ$C for 10 min. Then, 500 $\mu$l of a substrate solution (CM-amylose 6,0 mg/ml, TBS) was added dropwise and further reacted at 37 $^\circ$C for 10 min. The reaction was terminated by the addition of 1 ml of EDTA and, after adding 100 $\mu$l of xylidine, measurement was read at 620 nm. The results are shown in Table 1.

Table 1: Effect of α-amylase inhibitor obtained from soy-bean and wheat on the activity of various kinds of saccharase.

| Saccharases | α-amylase inhibitors (%)* | |
|---|---|---|
| | soy-bean | Wheat |
| Human saliva α-amylase | 90 | 93 |
| Mouse pancreatic α-amylase | 80 | 70 |
| Bacillus subtilis α-amylase | 80 | 70 |
| Bacillus subtilis ß-amylase | 63 | 50 |
| Yeast maltase** | 40 | 30 |
| Yeast invertase** | 80 | 47 |

*:   1 unit of α-amylase inhibitor derived from soy-bean or wheat was acted on 1 unit of each enzyme and the inhibitor activity was indicated by "%".

**:  Maltose was used as a substrate for maltase, while sucrose was used for the substrate of invertase and the activity was measured based on the amount of the resultant glucose.

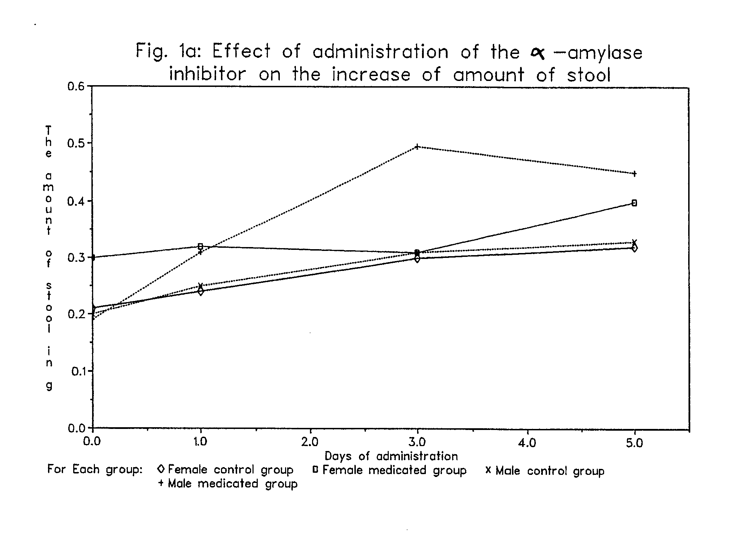

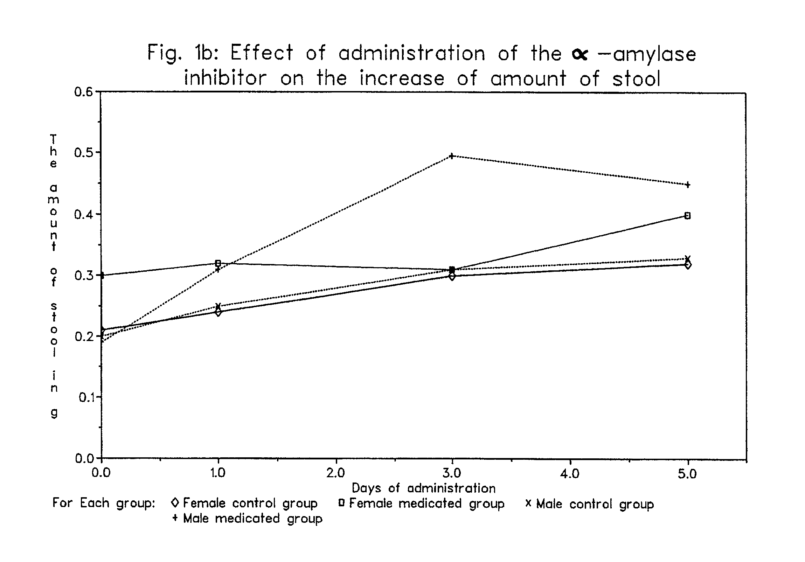

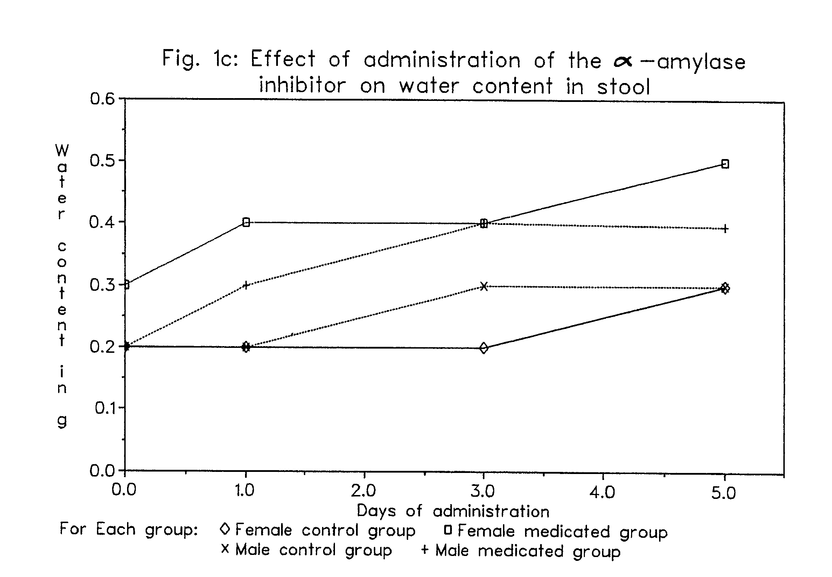

(2) Male and Female mice (ddY) of three week age were reared each by tens for one week on feed prepared by blending casein, corn starch and composite vitamins. α-amylase inhibitor derived from soy-bean was dissolved in tap water and administrated by 2 mg (2.780 units) / mouse per a day. The administration was made by free oral administration. As a control, male and female mice were reared each by 10 in the same manner and administrated with 2 mg / mouse of bovine serum albumin instead of α-amylase inhibitor. The amount of stool and the water contents in stool were examined and the body weight was also measured for the test group and the control group. The results are shown in Fig. 1a, 1b and 1c.

As shown in Fig. 1a, 1b and 1c, the effect of suppressing the body weight by the administration of the α-amylase inhibitor was remarkably shown from the next day after starting the administration. The maximum suppression rate for the increase of the body weight during the test period was as high as about 50 %. The amount of stool was increased by about 20 % and the water content in the stool was also increased somewhat.

(3) Male and female mice (ddY) of three week age were reared each by 10 in the same manner as in experiment 1 for one week and the behavior of α-amylase inhibitor in the digestive track during rearing was examined based on the remaining activity of α-amylase inhibitor. Further, since there was a speculation that α-amylase in blood is derived from saliva, change of α-amylase activity in the blood was examined. The results are shown in Table 2a.

Table 2a: Residual activity of α-amylase inhibitor in
digestive tracks.

| Digeative organs* | α-amylase inhibitor activity (%)** |
|---|---|
| Stomach | 85,4 ( 0,3) |
| Duodenum | 89,0 (12,5) |
| Upper portion in small intestine | 76,8 (10,3) |
| Middle portion in small intestine | 78,8 ( 9,4) |
| Lower portion in small intestine | 70,3 ( 8,9) |
| Colon | 80,7 (10,7) |

*:   The residual activity of the α-amylase inhibitor based on
     a predetermined amount of contents in each of the organs
     was examined.

**:  The rate of the residual activity was calculated assuming
     the activity of the administrated α-amylase as 100 %.

As shown in Table 2a, the α-amylase inhibitor of the present invention showed resistance to each proteases in gastric or pancreatic juice and about 70 % of the inhibitor activity was possessed in the lower portion of the small intestine. Further, since about 60 % of the activity was remained also in the colon, it was confirmed that the α-amylase inhibitor also showed resistance to the protease yielded from intestinal microorganisms.

Table 2b: Change of α-amylase content in blood

| Days of administration of α-amylase inhibitor | α-amylase activity* |
|---|---|
| 0 | 100 |
| 2 | 98,8 (5,0) |
| 4 | 102,4 (6,2) |
| 5 | 99,1 (5,5) |

*:   The α-amylase activity in blood before administration of
     the α-amylase inhibitor was assumed as 100 %.

As shown in Table 2b administration of the α-amylase inhibitor give no effect at all on the α-amylase activity in the blood.

(3a) While giving usual diet (about 2.400 Kcal) to a 38 years old man with 163 cm of height and 78,5 kg of body weight α-amylase inhibitor obtained from soy-bean was orally administrated by 100 mg (1.380.000 units) for one month during taking a supper. The body weight was measured before and after on every defecation and the difference was defined as an amount of stool. The results are shown in Table 3a.

**Table 3a. Variation of the body weight and the amount of stool caused by the administration of α-amylase inhibitor.**

| Days of admini- stration | body weight (kg) | amount of stool (kg) |
|---|---|---|
| 0 | 76,5 | 0,7 |
| 5 | 75,5 | 1,2 |
| 10 | 74,5 | 1,2 |
| 15 | 74,0 | 1,0 |
| 20 | 74,0 | 0,9 |
| 25 | 73,5 | 1,2 |
| 30 | 73,2 | 1,2 |

As shown in Table 3a, the body weight after one month was 73,2 kg and there was a meaningful increase in the amount of stool during the administration period of the α-amylase inhibitor.

(3b) While giving usual diet (about 2.500 Kcal), α-amylase inhibitor obtained from soy-bean was orally administrated for fast one month, then instead of the same amount of α-amylase inhibitor obtained from wheat to a 58 years old male with 180 cm of height and 93 kg of body in the same way as in experiment (3a). The body weight and the blood pressure during the administration period were measured in a rest state just after getting up. The results are shown in Table 3b.

Table 3b: Variation of the body weight and the blood pressure
of a male by the administration of α-amylase
inhibitor.

| Days of administration | body weight (kg) | arterial blood pressure(mmHg) | venus blood pressure(mmHg) |
|---|---|---|---|
| 0 | 93,5 | 166 | 112 |
| 5 | 93,0 | 162 | 116 |
| 10 | 92,5 | 150 | 90 |
| 15 | 92,5 | 143 | 108 |
| 20 | 91,0 | 151 | 108 |
| 25 | 90,0 | 144 | 98 |
| 30 | 90,5 | 148 | 96 |
| 35 | 90,0 | 152 | 102 |
| 40 | 90,0 | 156 | 110 |
| 45 | 89,5 | 134 | 94 |
| 50 | 89,5 | 144 | 98 |
| 55 | 89,5 | 142 | 92 |
| 60 | 89,5 | 148 | 96 |
| 65 | 89,5 | 156 | 98 |
| 70 | 89,0 | 140 | 92 |
| 75 | 89,0 | 140 | 92 |
| 80 | 86,5 | 152 | 98 |
| 85 | 88,5 | 148 | 92 |
| 90 | 88,0 | 145 | 92 |

As shown in Table 3b, decrease of the body weight during 3 months was 5 kg and there was a meaningful decrease both of the arterial and the venus blood pressure such as from 168 mmHg and 112 mmHg to 145 mmHg and 92 mmHg, respectively, during the administration period.

Both in experiments 3a and 3b, although the amount of stool tended to be increased during the period of oral administration of α-amylase inhibitor, no caddy stool or diarrhea, as well as unpleasant feeling relevant thereto were not recognized. In addition, no changes were recognized at all for the functions of liver, pancreas and kidney.

α-amylase inhibitor used in the previous experiments was obtained from soy-bean, but α-amylase inhibitor obtained from wheat has also a similar effect. The amount of using and the method of administrating α-amylase inhibitors are merely for the explanation of the invention but they do not particularly restrict α-amylase inhibitors for the origin, the amount of use, the administration method or the like.

As has been described above, the present invention can provide a remarkable effect for the cure and prevention of obesity by reducing the total calorie taken from food-stuff by restricting the digestion and absorption of starch.

**Claims**

1. A method of curing and preventing obesity comprising orally administrating α-amylase inhibitor in an amount effective to the purpose of use to a human or an animal, thereby inhibiting an α-amylase activity in saliva and pancreatic juice, and reducing the digestion and absorption of starch (reduction of the calorie taken from meals).

2. A method as defined in claim 1, wherein the α-amylase inhibitor used is obtained from beans.

3. A method as defined in claim 1, wherein the α-amylase inhibitor used is obtained from wheat.

4. A method as defined in claim 1, wherein the α-amylase inhibitor comprises polysaccharide and / or protein.

5. A method as defined in claim 1, wherein the α-amylase inhibitor is orally administrated in an optional form such as a liquid or solid depending on the purpose of use.

Fig. 1a: Effect of administration of the α −amylase inhibitor on the increase of amount of stool

For Each group: ◊ Female control group   □ Female medicated group   ✕ Male control group
+ Male medicated group

Fig. 1b: Effect of administration of the α –amylase inhibitor on the increase of amount of stool

For Each group:  ◊ Female control group   □ Female medicated group   × Male control group
+ Male medicated group

Fig. 1c: Effect of administration of the $\alpha$-amylase inhibitor on water content in stool

For Each group:  ◊ Female control group    □ Female medicated group
                 ✕ Male control group      + Male medicated group